# EUROPEAN PATENT APPLICATION

(11) **EP 1 990 030 A2**
(43) Date of publication of application: **12.11.2008**
(21) Application number: 08155909.8
(22) Date of filing: 08.05.2008
(51) Int. Cl.: A61F 7/03

(54) **Warming devices with elastic disposed therein and methods of warming**

(30) Priority: 08.05.2007 US 745850
(71) Applicant: Heatmax, Inc., Dalton, GA 30722 (US)
(72) Inventor: Yim, Daniel H., Rocky Face, GA 30740 (US); Ramachandran, Uma, Dalton, GA 30720 (US)
(74) Representative: Rehberg, Bernhard Frank

(57) **Abstract**

Self-contained disposable single-use heat-generating apparatuses and methods for providing heat are disclosed that have elastic member formed on at least a portion thereof. One exemplary apparatus includes a heat-generating pack having a first bag layer defined by a first surface area bonded to a second bag layer defined by a second surface area and creating one or a multiple pouches therebetween, an elastic member being disposed between at least two pouches. A heat-generating agent is disposed within the pouch(es) and adapted to consume air at a predetermined consumption rate in an exothermic reaction. At least a portion of one of the first surface area and the second surface area comprises an air permeable surface area having a predetermined airflow rate such that the heat-generating agent remains substantially evenly distributed within the pouches.

## Description

### TECHNICAL FIELD

The present disclosure is generally related to warming devices and, more particularly, is related to a self-contained personal warming apparatus and method of warming.

### BACKGROUND

Heat-generating pouches of various configurations and shapes are designed and used for various purposes, such as hand warming, feet warming, and the like, by placing the heat-generating pouch in a glove, mitten, shoe, etc. Heat-generating pouches typically include a heat-generating compound disposed between at least two layers of material, such as fabric, or the like, assembled to form a pouch. The heat-generating compound emits heat during an exothermic chemical reaction resulting from exposure of the compound to air. Known heat-generating compounds typically include a loose granular substance that is freely movable within the pouch. With a freely movable compound, when the pouch is placed flat, or horizontally, the compound is somewhat evenly distributed throughout the pouch. However, when the pouch is placed vertically, moved around, or jostled, the compound is drawn by gravity, shifts, and settles toward one end of the pouch. This shifting and settling of the compound is sometimes referred to as a "tea-bag" effect. The tea-bag effect results in an uneven temperature profile along the surface area of the pouch and produces an uncomfortable feeling for a user of the pouch. An uneven temperature profile can result in some areas not receiving heat, as desired, or an over concentration of heat in other areas.

The problem of the compound tending to shift and settle within the pouch has been addressed by other configurations of heat-generating pouches. In one embodiment, the heat-generating compound is contained within pucks or pellets that are disposed between at least two layers of material. The pucks or pellets comprise a heat-generating compound capable of reacting with air in an exothermic reaction. The compound is compressed into concentrated, substantially rigid, pellets. In this configuration, however, the heat emission is concentrated at the pucks, resulting in an uneven heat distribution across the surface area of the pouch. Furthermore, because the pucks are rigid, the pucks do not conform to various contours of the human body against which the heat-generating pouch may be placed.

The undesirable effect of a shifting compound has also been addressed by introducing air to the heat-generating compound through only one of the two layers of material forming the pouch, while the other of the two layers of material comprises a self-adhesive. However, these adhesive pouches cannot be easily inserted into pockets formed in socks, gloves, mittens, specially designed belts, or the like for use. Indeed, such adhesive pouches are typically fixed to an interior surface of a user's clothing. In this configuration of use, the pouch moves away from the user's skin as the clothing moves away from the user's skin. Furthermore, fixing the pouch to a user's clothing typically results in minimal or no pressure being applied to the pouch as the pouch is applied to the user's skin, thereby rendering the pouch less effective.

Prevailing medical knowledge is that in order to be considered therapeutic (e.g., for relief of muscle, joint, and/or menstrual cramp pain) a personal warming device emits heat to warm skin to a temperature range of about 39-45 °C. Adhesive that has been applied to cover all or substantially all of a layer of material forming the personal warming device that is applied to a user's skin results in constant, uninterrupted contact of the device with the skin. At the very least it can discomfort a user, and can even exceed the therapeutic temperature range and cause burns. Adhesive that only intermittently covers the surface area of the side of the warming device applied to a user's skin, however, can result in the edges of the warming device being lifted and the warming device inadvertently removed or peeled off the user's skin, such as when clothing engages an edge of the device and wedges under it. Adhesive applied only in tabs at each end of an elongated personal warming device (e.g., as in a back compress) can still result in the warming device from pulling away from a user's skin upon movement by the user.

Elastic members have been used to allow a personal warming device to conform to an individual's body contours, e.g., an elastic member that wraps around a user's waist, thereby securing the warming device to the user's back or stomach. These warming devices, however, typically include an elastic member that is bonded to the entire surface of the warming device touching the user's skin. Therefore, as the elastic members expand and shrink the heat profile of the heat emitted from the material covering the warming device is compromised.

Thus, a heretofore unaddressed need exists in the industry to address the aforementioned deficiencies and inadequacies.

### SUMMARY

One embodiment of the present disclosure provide a self-contained disposable single-use heat-generating apparatus and methods of providing therapeutic heat. Briefly described, one embodiment of the apparatus can be configured as follows. A self-contained disposable single-use heat-generating apparatus includes a heat-generating pack having a first bag layer bonded to a second bag layer creating a plurality of pouches therebetween. A heat-generating agent is disposed in each pouch. At least a portion of one of the first bag layer and the second bag layer has an air permeable surface area with a predetermined airflow rate, and the other of the first surface area and the second surface area comprises an air impermeable surface area with an elastic member disposed between at least two pouches. The airflow rate through the air permeable surface area is predetermined such that the heat-generating agent remains substantially evenly distributed within the pouches.

Other embodiments of the present disclosure can also be viewed as a method for providing therapeutic heat, including forming and/or using the heat-generating apparatus. In this regard, one embodiment of such a method, among others, can be broadly summarized by the following steps: containing a heat-generating composition in a plurality of pouches in a self-contained heat-generating pack and introducing air to the heat-generating composition such that the heat-generating composition remains substantially evenly distributed within the heat-generating pack, and providing an elastic member between at least two of the pouches of the heat-generating pack.

This method may further comprise forming a plurality of pouches in the heat-generating pack by forming seams in the heat-generating pack, wherein the elastic member is disposed on at least one seam of the heat-generating pack, between two pouches.

The elastic member may be attached to the two pouches by at least one of the following methods: heat lamination, pressure lamination, gluing, sewing, snapping, and combinations thereof.

The method may further comprise the steps of: providing adhesive on only a portion of one outside air impermeable surface of the heat-generating pack, wherein the surface has a low coefficient of friction.

The step of providing the adhesive on only a portion of one outside air impermeable surface of the heat-generating pack may comprise providing the adhesive over an outer surface of the pouch.

The heat-generating pack may be used for therapeutic purposes, and the heat generated from the pack may have a therapeutic temperature range.

Other systems, methods, features, and advantages of the present disclosure will be or become apparent to one with skill in the art upon examination of the following drawings and detailed description. It is intended that all such additional systems, methods, features, and advantages be included within this description, be within the scope of the present disclosure.

### BRIEF DESCRIPTION OF THE DRAWINGS

Many aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.
- FIG. 1: is a cutaway perspective view of an embodiment of the self-contained personal warming apparatus of the present disclosure.
- FIG. 1A: is a perspective view of an embodiment of a self-contained personal warming apparatus illustrated in FIG. 1.
- FIG. 2: is a plan view of an embodiment of a bag layer of the apparatus illustrated in FIG. 1.
- FIG. 3: is a plan view of an embodiment of a bag layer of the apparatus illustrated in FIG. 1.
- FIG. 4: is a plan view of an embodiment of a bag layer of the apparatus illustrated in FIG. 1.
- FIG.5: is a plan view of an embodiment of the disclosed self-contained personal warming apparatus.
- FIG.6: is a plan view of an embodiment of the disclosed self-contained personal warming apparatus.
- FIG.7: is a plan view of an embodiment of the disclosed self-contained personal warming apparatus.
- FIG. 8: illustrates a plan view of an embodiment of the disclosed self-contained personal warming apparatus.
- FIG. 9: illustrates a cross sectional view of an embodiment of the disclosed self-contained personal warming apparatus taken along section line A-A in FIG. 8.
- FIG. 10: illustrates a cross sectional view of an embodiment of the disclosed self-contained personal warming apparatus taken along section line A-A in FIG. 8.
- FIG. 11: illustrates a plan view of an embodiment of the disclosed self-contained personal warming apparatus.
- FIG. 12: illustrates a cross sectional view of an embodiment of the disclosed self-contained personal warming apparatus taken along section line B-B in FIG. 11.

### DETAILED DESCRIPTION

It is desirable to utilize one or more elastic members on at least a portion of a heat-generating apparatus in order to conform the apparatus to a user's body contours. It is preferable to apply the elastic member(s) to discrete portions of the apparatus, and not as a backing underneath the entire surface of the apparatus that is touching the user's skin.

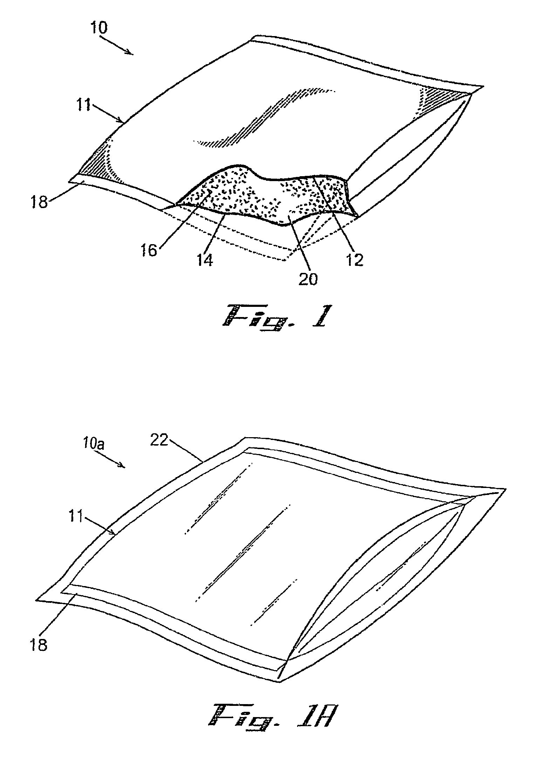

FIG. 1 illustrates one preferred embodiment of a self-contained disposable single-use heat-generating apparatus 10 of the present disclosure. A heat-generating pack 11 comprises a first bag layer 12, a second bag layer 14 and a heat-generating agent 16 disposed therebetween. The first bag layer 12 is defined by a first set of dimensions and has a first surface area. The second bag layer 14 is defined by a second set of dimensions and has a second surface area. It should be noted that although the dimensions of the first bag layer 12 and the second bag layer 14 are illustrated as being substantially rectangular in shape, the dimensions can form any suitable shape. The first surface area can substantially correspond to the second surface area.

The first bag layer 12 and the second bag layer 14 are aligned, one on top of the other, and are fixed together by at least one seam 18. The seam 18 can either extend around the perimeter of the heat-generating pack 11 where the first bag layer 12 and second bag layer 14 meet, or run along one or a plurality of edges thereof. As used herein, the term "plurality" can be both "several" and/or "more than one" (*i*.*e*., two). As illustrated in FIG. 1, the seam 18 runs along two opposing edges. The seam 18 is created in any suitable manner, for example by melting, bonding, or sewing.

At least one enclosed space, or pouch 20, is created between the first bag layer 12 and the second bag layer 14. Although only one enclosed space 20 is shown in FIG. 1, as discussed later, multiple pouches 20 can be formed in the heat-generating pack 11. At least a portion of one of the first surface area and/or the second surface area is/are preferably air permeable as discussed in greater detail below. The first bag layer 12 and the second bag layer 14 preferably comprise a flexible fabric, material, or the like.

A heat-generating agent 16 is disposed within the pouch 20 and contained therein. The heat-generating agent 16 comprises a main ingredient of iron powder and incorporates therein water, a water retaining material (e.g., charcoal, vermiculite, or the like), an oxidation promoter, such as activated carbon, and salt. More particularly, and as an example, the agent 16 may comprise approximately 35-50% by weight of iron powder, about 25-45% by weight of water, approximately 10-14% by weight of water retaining agent, and approximately 4.5-6% by weight of salt. Upon exposure to air, oxidation of the iron begins in an exothermic reaction. The heat generated by the exothermic reaction of the agent 16 passes through the first bag layer 12 and the second bag layer 14 and radiates from the apparatus 10. It is preferable that the heat radiating from the apparatus 10 warms skin to a temperature range from about 39-45°C in order to provide a level of heat suitable for therapeutic heating.

During the exothermic reaction, the heat-generating agent 16 consumes air at a predetermined air consumption rate. Controlling the rate of introduction of air to the heat-generating agent 16 affects both the temperature radiated from the pack 11, as well as the shifting of the agent 16 within the pouch 20. Generally, the more air introduced to the heat-generating agent 16, the hotter the pack 11 will become. Also, where the heat-generating agent 16 consumes air faster than air is introduced to thereto, a vacuum will be created. Thus, the heat-generating agent 16 can remain in place in the pouch 20 through the use of differential pressure.

More specifically, and with reference to FIG. 1A, an embodiment of the self-contained disposable single-use heat-generating apparatus 10a is illustrated. In this embodiment, the heat-generating pack 11 is disposed inside a protective package 22. The protective package 22 can be hermetically sealed with the heat-generating pack 11 inside such that no air or minimal air is introduced to the heat-generating pack 11. In this embodiment, the protective package 22 effectively eliminates the introduction of air to the agent 16 thereby substantially preventing the heat-generating exothermic reaction. The heat-generating pack 11 is disposed within the protective package 22 preferably at, or closely after, the time of manufacture, and the heat-generating apparatus 10 can be marketed, sold, and stored in this configuration.

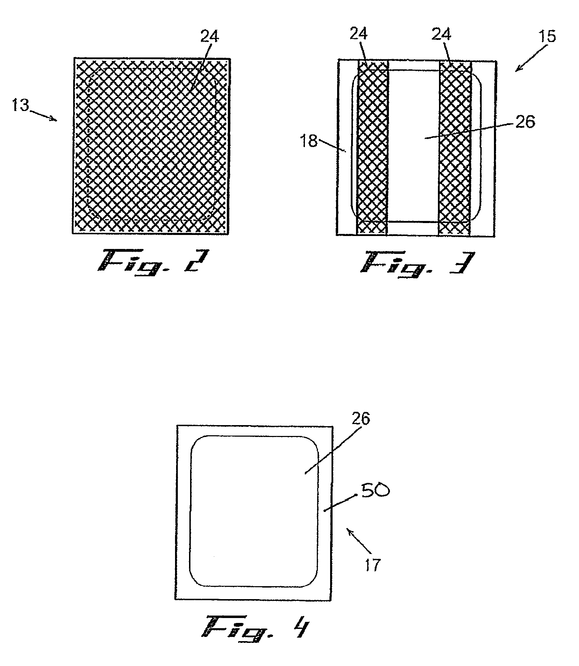

Referring next to FIGs. 2-4, various embodiments of bag layers 13, 15, and 17 are illustrated. The bag layers 13, 15, and 17 can comprise the first bag layer 12, the second bag layer 14 or any suitable combination thereof in order to form a heat-generating pack 11. For example, a heat-generating pack 11 can comprise a first bag layer 12 arranged in the configuration of the bag layer 13 (FIG. 2) and a second bag layer 14 arranged in the configuration of the bag layer 17 (FIG. 4).

Selection of the configuration of first bag layer 12 and second bag layer 14 is driven by a desired airflow rate for introduction of air to the heat-generating agent 16. An air consumption rate of the heat-generating agent 16 being at least slightly greater than an airflow introduction rate to the agent 16 generates at least a slight vacuum inside the pouch 20. The vacuum created inside the pouch 20 reduces shifting and settling of the heat-generating agent 16, or "tea-bagging," within the pouch 20.

The substantially stationary disposition of the heat-generating agent 16 inside the pouch 20 results in a heat-generating pack 11 that maintains a substantially constant thickness. A substantially even heat profile is emitted across the surface area of the first bag layer 12 and the second bag layer 14. The airflow rate through the combined first surface area and second surface area of the first bag layer 12 and the second bag layer 14 preferably is less than the predetermined air consumption rate of the heat-generating agent 16 during exothermic reaction. For example, a heat-generating pack 11 having porosity allowing an airflow rate of about 20,000 sec/100 cc of air preferably contains a heat-generating agent 16 having an air consumption rate greater than about 20,000 sec/100cc of air during the exothermic reaction.

Referring more specifically to FIG. 2, one embodiment of the bag layer 13 configuration comprises an air permeable surface area 24. The air permeable surface area 24 preferably comprises a microporous fabric. A preferred microporous fabric can comprise a nonwoven fabric formed from individual fibers that are pressed together forming an interlocking web of fibers. The fibers can be fixed to each other either mechanically (for example, by tangling the fibers together) or chemically (for example, by gluing, bonding, or melting the fibers together). The disclosed heat-generating pack 11 can comprise a microporous fabric known to one having ordinary skill in the art.

FIG. 3 illustrates another embodiment of a bag layer 15 configuration having a portion of the surface area thereof comprising an air permeable surface area 24 and a portion of the surface area comprising an air impermeable surface area 26. In one embodiment, all surface areas of the bag layer 15 can be of a material of low permeability, so long as differential pressure is created between surface areas 24, 26.

The air permeable surface area 24 preferably comprises a microporous fabric. A preferred microporous fabric for this configuration can comprise a nonwoven fabric formed from individual fibers that are pressed together forming an interlocking web of fibers. The fibers can be fixed to each other either mechanically (for example, by tangling the fibers together) or chemically (for example, by gluing, bonding, or melting the fibers together). This configuration can comprise a microporous fabric known to one having ordinary skill in the art. The air impermeable surface area 26 of the bag layer 15 can include polyethylene, polypropylene, or any suitable material. In one embodiment, the air impermeable surface area 26 exhibits a low coefficient of friction, such as to allow the heat-generating pack 11 to easily slide into a pocket (not shown) formed in a glove, sock, belt for holding heat-generating packs in position, or the like. The preferred combination of air permeable surface area 24 and air impermeable surface area 26 of the bag layer 15 of FIG. 3 is determined by the desired air flow introduction rate to the heat-generating agent 16 inside a pouch 20 that this bag layer 15 configuration can be used to form. In other embodiments, the air impermeable surface area 26 does not have a low coefficient of friction.

FIG. 4 illustrates another embodiment of a bag layer 17 configuration. The bag layer 17 comprises an air impermeable surface area 26, such as polyethylene, or any suitable material. It is preferable that the air impermeable surface area 26 exhibits a low coefficient of friction, such as to allow the heat-generating pack 11 to easily slide into a pocket (not shown) formed in a glove, sock, belt for holding heat-generating packs in position, or the like.

An adhesive can be applied to a portion of the air impermeable surface area 26 of the bag. In one embodiment, the adhesive is applied at least to the perimeter 50 of an outside surface of the impermeable surface area that faces a user's skin. In one embodiment, the adhesive is applied to selected portions of the pack 11 in a manner whereby the pack 11 is sufficiently attached to a user's skin to allow movement by the user without dislocating a majority of the pouch(es) 11 from contact with the skin. The adhesive is not applied to the entire impermeable surface area 26 for a personal warming device used for therapeutic purposes (e.g., where the user's skin is warmed to about 39-45 °C).

In one embodiment the adhesive is a composition suitable for attaching the heat-generating pack 11 to a user's skin whereby the pack 11 can be peeled off or removed from the skin after use. Adhesives that are suitable for the disclosed heat-generating packs 11 include synthetic elastomers suitable for attachment of the heat-generating pack 11 to a user's skin and then removal after use. In one embodiment, the adhesive transmits heat from the pack 11 to the user's skin. An example of a suitable adhesive includes a double-coated adhesive tape. The double-coated tape can include two (2) sides coated with same on different adhesives. The adhesive on the side of the tape facing the pack 11 can be, for example, a synthetic latex adhesive. The adhesive on the side of the tape facing a user's skin can be a medical-grade and/or hypoallergenic tape, such as, but not limited to, an acrylate-based adhesive. An exemplary double-coated adhesive tape is commercially available from 3M Inc. of St. Paul, Minnesota, USA. Adhesives that are suitable for the heat-generating pack 11 also include those disclosed in U.S. Patent No. 6,177,482 to Cinelli et al*.,* incorporated herein by reference in its entirety.

The adhesive can be applied to the heat generating pack by, for example, spraying, deposition by a drop-on-demand device (e.g., an ink-jet device), painting, rolling, taping, etc. In one embodiment of the heat-generating pack 11, the adhesive has applied thereto a releasable liner for protection of the adhesive prior to application of the heat-generating pack to a user's skin.

Applying the above disclosed bag layer configurations 13, 15 and 17, heat-generating packs 11 of various configurations can be formed. One configuration of a heat-generating pack 11 comprises a first bag layer 12 comprising bag layer 13 configuration having an air permeable surface area 24 (illustrated in FIG. 2) and a second bag layer 14 comprising the bag layer 17 having an air impermeable surface area 26 (illustrated in FIG. 4). In this configuration, the rate at which air is introduced to the heat-generating agent 16 is controlled by allowing a predetermined flow rate through the first bag layer 12 and allowing substantially no air flow through the second bag layer 14.

Another configuration of a heat-generating pack 11 comprises a first bag layer 12 comprising the bag layer 13 having an air permeable surface area 24 (illustrated in FIG. 2) and a second bag layer 14 also comprising the bag layer 13 also having an air permeable surface area 24 (illustrated in FIG. 2). In this configuration, the rate at which air is introduced to the heat-generating agent 16 is controlled by allowing a pre-determined flow rate through both the first bag layer 12 and the second bag layer 14.

An embodiment of the disclosed heat-generating pack 11 can also comprise a first bag layer 12 comprising the bag layer 13 having an air permeable surface area 24 (illustrated in FIG. 2) and a second bag layer 14 comprising the bag layer 15 having a portion of the surface area being an air permeable surface area 24 and a portion of the surface area being an air impermeable surface area 26 (illustrated in FIG. 3). In this configuration, the rate at which air is introduced to the heat-generating agent 16 is controlled by the total air permeable surface area 24 of the first bag layer 12 and the second bag layer 14 combined. It is preferable that the airflow rate through the total air permeable surface area 24 of the first bag layer 12 and the second bag layer 14 combined is less than the air consumption rate of the heat-generating agent 16 during exothermic reaction.

An embodiment of the disclosed heat-generating pack 11 can also comprise a first bag layer 12 comprising bag layer 17 having an air impermeable surface area 26 (illustrated in FIG. 4) and a second bag layer 14 comprising bag layer 15 having a portion of the surface area being an air permeable surface area 24 and a portion of the surface area being an air impermeable surface area 26 (illustrated in FIG. 3). In this configuration the rate at which air is introduced to the heat-generating agent 16 is controlled by the total air permeable surface area 24 of the second bag layer 14. It is preferable that the airflow rate through the total air permeable surface area 24 of the second bag layer 14 combined is less than the air consumption rate of the heat-generating agent 16 during exothermic reaction.

It should be noted that the above described heat-generating packs 11 are mere examples and that any configuration combining air permeable surface area 24 with air impermeable surface area 26 is contemplated by the present disclosure.

In one method of use of an embodiment of the disclosed self-contained disposable single-use heat-generating apparatus 10, a heat-generating pack 11 is disposed in a protective package 22 to eliminate, or at least minimize, introduction of air to the heat-generating agent 16 disposed inside the pack 11. The heat-generating pack 11 is removed from the protective package 22. Air is introduced to a heat-generating agent 16 disposed within a pouch 20 of the heat-generating pack 11. The pouch 20 is formed by a first bag layer 12 and a second bag layer 14 being peripherally bonded to each other. The heat-generating agent 16 consumes air in a heat-generating exothermic reaction, thereby emitting heat from the heat-generating pack 11. At least one of the first bag layer 12 and the second bag layer 14, or a combination thereof, allow air to be introduced to the heat-generating agent 16. The introduction of air is preferably at a flow rate less than the air consumption rate of the heat-generating agent 16 during the exothermic reaction. The heat-generating pack 11 can be positioned, as desired.

In one method of use, the heat-generating pack 11 can be inserted into a pocket, for example a pocket disposed in a belt for heat application near a user's skin on their back, stomach, or any desired location. The heat-generating pack 11 can also be inserted into a pocket formed in a sock or glove for a user to warm toes and fingers, respectively.

The exothermic reaction of the heat-generating agent 16 when introduced to air produces a therapeutic heat emission for approximately 12 to 18 hours. Upon the conclusion of the exothermic reaction and the cooling down of the heat-generating pack 11, the heat-generating pack 11 can be removed from the position at which it was placed for use and disposed.

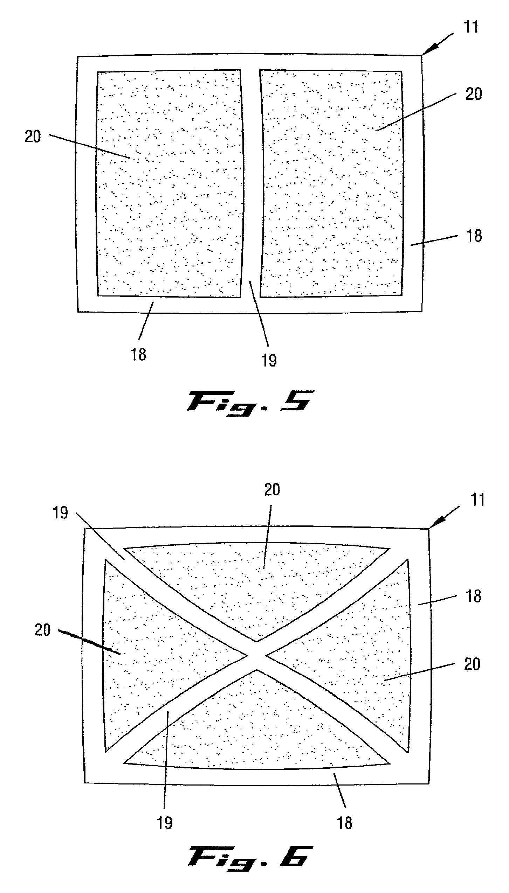

As noted above and demonstrated in FIG. 5, multiple pouches 20 can be formed in the heat-generating pack 11. In one exemplary configuration, the first bag layer 12 and the second bag layer 14 are fixed together or joined at multiple seams, such as a first seam 18 and a second seam 19 shown in FIG. 5. In one such embodiment, the first seam 18 and the second seam 19 compartmentalize the heat-generating pack 11 into separate heat-generating pouches 20. In the embodiment depicted, the first seam 18 extends around the perimeter of the heat-generating pack 11. The second seam 19 extends between two separate pouches 20. In one embodiment, the first seam 18, extending around the perimeter of the pack 11, has adhesive disposed thereon. In one embodiment, the adhesive extends all the way to the edge of the pack 11. In one embodiment, both the first seam 18 and the second seam 19 have adhesive disposed thereon, extending around the perimeter of the pack and through a middle portion thereof.

The first seam 18 and the second seam 19 can be formed in the same or a different manner. For example, the first seam 18 can be formed first, followed by the formation of the second seam 19. Alternatively, both the first seam 18 and the second seam 19 can be created by, for example, melting both seams at the same time. Even though one particular configuration has been shown in FIG. 5 for the first seam 18 and the second seam 19, one can envision other embodiments of a multi-seamed pack 11, for example in a criss-cross shape (as illustrated in FIG. 6), or multiple vertical and/or horizontal seams.

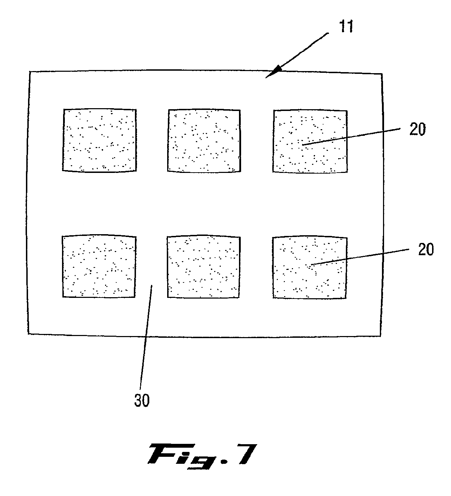

Alternatively, multiple pouches 20 can be formed from the first bag layer 12 and the second bag layer 14 as shown in FIG. 7. The pouches 20 can be smaller in size and can be formed more as pockets in the heat-generating pack 11. In this manner, areas 30 are formed in the pack 11 whereby the first bag layer 12 and the second bag layer 14 are touching in some manner to prevent shifting of the heat-generating agent from one pouch 20 to another. In one embodiment, the areas 30 extending between the pouches 20 have adhesive applied thereto. In one embodiment, the areas 30 have adhesive applied thereon only around the perimeter, and extending all the way to the edge of the heat-generating pack 11. The pouches 20 illustrated in FIG. 7 can be formed by discrete seams around each pouch, or by generally melting or bonding the first bag layer 12 to the second bag layer 14 to bonded areas 30.

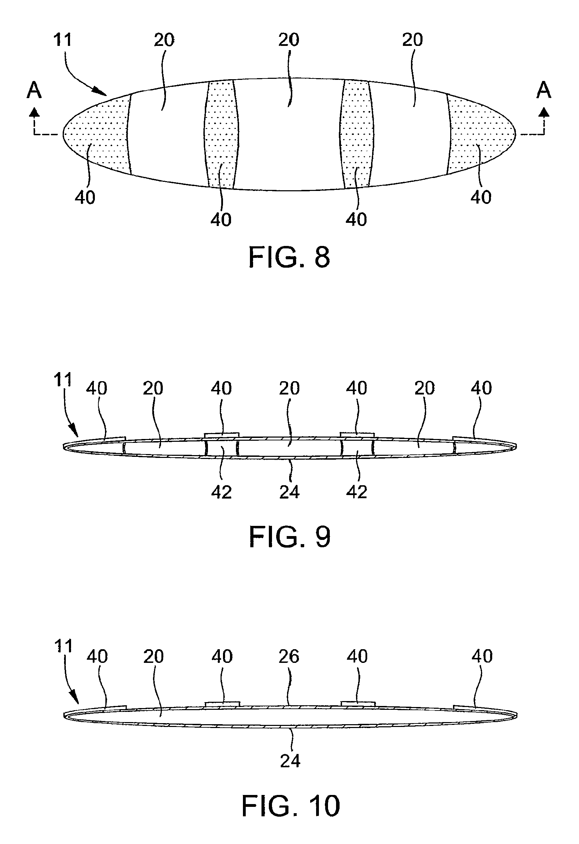

FIG. 8 is a simplified top view illustration of an embodiment of the disclosed pack 11 with adhesive areas 40 disposed between the heat pouches 20. FIG. 9 illustrates a cross-sectional view of an embodiment of the heat pouch 11 taken along section lines A-A of FIG. 8. In the embodiment of FIG. 9, the pack 11 has a plurality of large heat pouches 20 disposed between, but not under the adhesive areas 40. Seams 42 run under the adhesive areas 40. As can be seen in FIG. 9, the air permeable layer 24 is on the side opposite the adhesive areas 40, and therefore does not contact a user's skin.

FIG. 10 depicts a cross-sectional view of an embodiment of the heat-generating pack 11 taken along section lines A-A of FIG. 8. In the embodiment of FIG. 10, the pack 11 has a single large heat pouch 20 disposed underneath the adhesive areas 40. An alternative embodiment may combine the features of FIGs. 9 and 10, whereby adhesive areas 40 are disposed directly over both heat pouches 20 and/or seams 42, so long as the adhesive areas 40 do not cover the entire air impermeable surface 26. It should be noted that in each of embodiments FIGs. 8-10 the heat generating agent 16 is relatively uniformly disposed through the pouch(es) 20 and does not form pucks or discs of heat generating agents. Thus, the heat profile emitted from the heat-generating pouch(es) is relatively uniform across each pouch 20.

As discussed previously, the heat-generating pack 11 can comprise one or more elastic members. The elastic members can include natural or synthetic rubber, or any number of polymeric materials that are capable of elongation and recovery. Suitable materials include, but are not limited to, styrene block copolymers, rubber, Lycra^{™}, Krayton^{™}, polyethylene including metallocene catalyst polyethylene (PE), foams including polyurethane and polyesters, and the like, and combinations thereof. The elastic members can be in the form of films, strands, scrims, ribbons, tapes, structural elastic-like film, and the like, and combinations thereof. A particularly suitable material for use as the elastic members is an elastic scrim available as T50018 from Conwed Plastics, Minneapolis, Minnesota, USA.

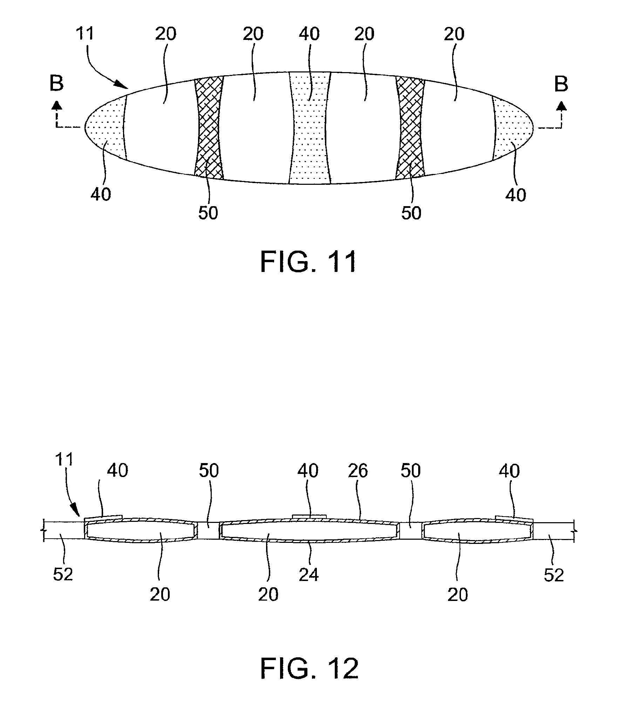

Depicted in FIGs. 11 and 12 are embodiments of the heat-generating packs 11 with elastic members 50. The elastic members 50 are disposed in strips between individual pouches 20. The number of elastic members can differ from that shown in FIG. 11. Optionally, adhesive areas 40 can be disposed on the pack 11. The adhesive areas 40 can cooperate with the elastic members 50 to keep the pack 11 close to a user's skin without adhering the entire pack 11 completely to the user's skin. The optional adhesive areas 40 can be configured in a different arrangement than that shown in FIG. 11.

FIG. 12 depicts a cross-sectional view of an embodiment of the heat-generating pack 11 taken along section lines B-B of FIG. 11. In the embodiment of FIG. 12, the pack 11 has elastic members 50 bonded to the pouches 20. The elastic members 50 can have a top side, an under side, and a left and right side. In one embodiment, the under side of the elastic member 50 faces the user's skin. In one embodiment, the elastic members 50 are bonded to the pouches 20 on both the left and right sides of the elastic members 50. In one embodiment, one or more, e.g., a pair of the elastic members 50, extend from one or more sides or ends of the heat-generating pack. In this embodiment, the elastic members can be elongated and be configured to stretch over or around at least one body part. Further, each pair of elastic member can include a fastening means configured to coincide with the fastening means on the other elongated elastic member. The fastening means can be, for example, hook and loop fasteners, a snap, a button, a zipper, a releasable adhesive, and combinations thereof. It should be noted that the elastic members 50 do not serve as a backing for the pouches 20, and are not co-extensive with the entire air permeable layer 24 or the air impermeable layer 26 of the pouches 20. The elastic members 50 can be attached to the air permeable layer 24 and/or the air impermeable layer 26 via, for example, heat lamination, pressure lamination, sewing, snaps, adhesive, etc.

In one embodiment, the pack 11 can include optional elastic members 52 that extend from one or both sides of the pack 11, allowing the pack 11 to be attached to a body part (e.g., wrapped around a user's leg, knee, midsection, etc.). In this embodiment, the elastic members 52 can include a fastening means, such as, but not limited to, hook and loop fasteners, snaps, buttons, zippers, releasable adhesive, etc. to secure the elastic members to each other.

In one embodiment of the disclosed heat-generating apparatus 10, the pouches 20 can include one or more scented compositions. As the heat-generating agent 16 emits heat, the scented substances in the pouches 20 will emit a stronger fragrance with the heat. The scent can be, for example, but not limited to one or more of the following: fruits, flowers, herbs, spices, or combinations thereof.

It should be emphasized that the above-described embodiments of the present disclosure are merely possible examples of implementations, and are merely set forth for a clear understanding of the principles herein. Many variations and modifications may be made to the above-described embodiment(s) without departing substantially from the spirit and principles of the disclosure. All such modifications and variations are intended to be included herein within the scope of this disclosure.

## Claims

1. A self-contained, disposable, single-use heat-generating apparatus, comprising:
a heat-generating pack comprising:
a first bag layer having a first surface area;
a second bag layer having a second surface area, the second bag layer being fixed to the first bag layer, such that the first bag layer and the second bag layer define a plurality of pouches therebetween;
a heat-generating agent disposed in each pouch, the heat-generating agent arranged and configured to consume air at a predetermined air consumption rate in an exothermic reaction;
at least one of the first surface area and the second surface area comprises an air permeable surface area having a predetermined airflow rate at which air is introduced to the heat-generating agent, the predetermined airflow rate being arranged and configured to be less than the predetermined air consumption rate such that the heat-generating agent remains substantially evenly distributed within the pouch; and
an elastic member fixedly attached to at least one of the first surface area and the second surface area, the elastic member extending between two individual pouches.

2. The apparatus of claim 1, wherein the other of the first surface area and the second surface area comprises an air impermeable surface area with an adhesive disposed on at least a portion of an outside surface of the air impermeable surface area, whereby the adhesive is suitable for attaching the heat-generating pack to a user's skin.

3. The apparatus of claim 1 or 2, wherein at least one of the first bag layer and the second bag layer comprises a microporous material, wherein the microporous material comprises a fabric having a plurality of fibers forming an inter-locking web, wherein at least a portion of the plurality of fibers are bonded to each other.

4. The apparatus of at least one of claims 1 to 3, wherein the elastic member comprises a material chosen from at least one of the following materials that are capable of elongation and recovery: natural rubber, synthetic rubber, polymeric materials, and combinations thereof.

5. The apparatus of at least one of claims 1 to 4, wherein the elastic member is in the form of at least one of the following types of materials: films, strands, scrims, ribbons, tapes, structural elastic-like film, and combinations thereof.

6. The apparatus of at least one of claims 1 to 5, further comprising:
a plurality of elastic members, wherein each member is disposed between individual pouches.

7. The apparatus of at least one of claims 1 to 6, wherein the elastic member is bonded to at least one of the following: the first surface area and the second surface area, whereby the elastic member is not co-extensive with the entire length of the surface area to which it is bonded.

8. The apparatus of at least one of claims 1 to 7, wherein the elastic member is attached to the pouches via at least one of the following: heat lamination, pressure lamination, gluing, sewing, snapping, and combinations thereof.

9. The apparatus of at least one of claims 1 to 8, further comprising a pair of elongated elastic members extending from at least two ends of the heat-generating pack.

10. The apparatus of claim 9, wherein the pair of elongated elastic members are configured to stretch over or around at least one body part.

11. The apparatus of claim 9, wherein each of the pair of elongated elastic members comprises a fastening means configured to coincide with the fastening means on the other elongated elastic member.

12. The apparatus of claim 11, wherein the fastening means comprises at least one of the following: hook and loop fasteners, a snap, a button, a zipper, a releasable adhesive, and combinations thereof.

13. The apparatus of at least one of claims 1 to 12, wherein the elastic member is configured to allow the heat-generating pack to stretch over or around at least one body part.

14. The apparatus of at least one of claims 1 to 13, wherein the plurality of heat-generating pouches comprise a substantially constant thickness, whereby a substantially even heat profile is emitted across the surface area of the first bag layer and the second bag layer.

15. The apparatus of at least one of claims 1 to 4, further comprising a scented composition disposed in the pouches.
